# EUROPEAN PATENT APPLICATION

(11) **EP 4 295 815 A1**
(43) Date of publication of application: **27.12.2023**
(21) Application number: 22819435.3
(22) Date of filing: 01.06.2022
(51) Int. Cl.: A61F 2/24

(54) **ROTATABLY ADJUSTABLE POSITIONING DEVICE**

(30) Priority: 10.06.2021 CN 202110647777
(71) Applicant: Jenscare Scientific Co., Ltd, Ningbo, Zhejiang 315336 (CN)
(72) Inventor: LV, Shiwen, Hangzhou Bay New District Ningbo, Zhejiang 315336 (CN); ZHENG, Linghe, Hangzhou Bay New District Ningbo, Zhejiang 315336 (CN); LING, Zhengqing, Hangzhou Bay New District Ningbo, Zhejiang 315336 (CN)
(74) Representative: Grünecker Patent- und Rechtsanwälte PartG mbB
(86) International application number: PCT/CN2022/096671
(87) International publication number: WO 2022/257837

(57) **Abstract**

Provided is a rotatably adjusted positioning device. The rotatably adjusted positioning device includes a fixing member (2), a steering mechanism (1), and at least one steering line (3). The connection mechanism (1) is substantially extending in a longitudinal direction and is rotatably connected to a distal side of the fixing member (2). The fixing member (2) is provided with a guide channel (21). One end of the steering line (3) is connected to the connection mechanism (1), and another end is connected to a steering mechanism (5) through a corresponding guide channel (21). The steering line (3) is operated by the steering mechanism (5) to make the connection mechanism (1) rotate relative to the fixing member (2), achieving an adjustment of a circumferential position of the connection mechanism (1) and components associated with the connection mechanism (1). The angle formed between the connection end (16) and the guide channel (21) on the cross section of the fixing member (2) can be adjusted by a pull of the steering line (3). Most of the pull force is concentrated between the guide channel (21) and the connection end (16). The loss is extremely small, and feedback on the adjustment is very timely and accurate.

## Description

This application claims priority to Chinese Patent Application No. 202110647777.7 filed with the China National Intellectual Property Administration (CNIPA) on Jun. 10, 2021 and titled ROTATABLY ADJUSTABLY POSITIONING DEVICE, the disclosure of which is incorporated herein by reference in its entirety.

### TECHNICAL FIELD

The present application relates to the field of medical devices and, in particular, to a rotatably adjusted positioning device.

### BACKGROUND

As one of the common valvular diseases, Aortic Stenosis (AS), with an incidence rate of 4.6% in people over 75 years of age in the United States, is the third most common cardiovascular disease in the United States following coronary heart disease and hypertension. Surgical aortic valve replacement has long been the only treatment recognized as effective in the long term. Nevertheless, based on high-risk assessments of surgical procedures and concerns about postoperative complications, 1/3 to 2/3 of patients give up surgical treatment. Therefore, once symptoms appear, the patient mortality rate averages 50% to 60% per year. With the continuous innovation of cardiac interventions and medical devices, medical catheterization, in particular, percutaneous aortic valve replacement (PAVR), has gradually become the mainstream procedure. Clinical trials have proved that PAVR is simple and feasible, bringing good news to many patients who cannot receive surgical treatment.

The current percutaneous aortic valve replacement has three main surgical approaches, namely, the forward technique (transfemoral vein and atrial septal puncture), the retrograde technique (reverse access to the aortic arch through the femoral artery), and the off-pump direct pathway valve replacement technique (transapical), the second of which is the most convenient and quickest and is widely used. Existing stent positioning devices in the market are represented by the Edwards balloon-expandable SAPIEN valve stent positioning device and the Corevalve self-expanding ReValving valve stent positioning device. R&D personnel continue to provide technical solutions and device innovations to increase the survival rate of patients and improve patients' living conditions.

Nevertheless, PAVR still has many deficiencies and technical problems that have not been solved or overcome in terms of aspects such as the selection of target population, long-term efficacy, and postoperative complications. Studies have shown that improvements in valve stent positioning devices and operation techniques play a critical role in inhibiting complications such as aortic perforation, perivalvular leakage, thrombosis, and stroke. For example, in patent CN201110092241.X, Edwards Lifesciences provides a heart valve positioning device (10) in which a prosthetic valve (16) is mounted on a valve catheter (23) inside a delivery sleeve (24). A stage balloon (18) protrudes from the delivery sleeve and provides a tapered surface for facilitating advancement through human vessels. The stage balloon also facilitates passage through the leaflets of the native valve. After the prosthetic valve is positioned within the native valve, the delivery sleeve is retracted to expose the prosthetic valve. In an embodiment, the delivery sleeve is retracted by using a lead screw (500) that achieves relative movement between the valve catheter and the delivery sleeve. The prosthetic valve is preferably self-expanding. If desired, the staged balloon may be expanded to securely place the prosthetic valve at the native valve site. The prosthetic valve is preferably connected to the valve catheter by a plurality of flexible extension arms (80) which enable the prosthetic valve to shrink after initial deployment of the prosthetic valve so that the prosthetic valve can be repositioned if desired. A deficiency of the technical solution of this existing art is that when the prosthetic valve enters the heart and is released for positioning, it is necessary to adjust the circumferential position of the artificial valve so that the prosthetic valve can be positioned more accurately. In this existing art, however, there is no design in which the prosthetic valve can be adjusted in the circumferential position. As a result, the prosthetic valve, during positioning, cannot be adjusted in the positioning position, and the prosthetic valve must be positioned in place in one step when the prosthetic valve is positioned in the heart. In this manner, the fault tolerance rate is very low, and the risk factor of the procedure is greatly improved.

As described above, existing aortic valve replacement procedures and devices do not have a design that enables the valve prosthesis to be adjusted in the circumferential direction after the valve prosthesis releases a positioning member in the heart. Therefore, it cannot be ensured that the positioning member can be more accurately inserted into the sinus during positioning to reduce the surgical risk. Therefore, there is an urgent need in the art for a positioning system for implanting devices such as valves. Such a positioning system features simpler operation, more precise positioning, fewer surgical complications, and shorter operating time.

### SUMMARY

In view of the preceding and other ideas, the present application is proposed. One of the purposes of the present application is to overcome the deficiencies of the existing art and provide a novel and rotatably adjusted positioning device for patients with cardiovascular diseases such as aortic stenosis who need interventional treatment.

According to another aspect of the present application, a rotationally adjustable positioning device is provided, including a fixing member, a control mechanism, and at least one steering line. The connection mechanism is substantially extending in a longitudinal direction and is rotatably connected to a distal side of the fixing member. The fixing member is provided with a guide channel. One end of the steering line is connected to the connection mechanism, and another end is connected to a steering mechanism through a corresponding guide channel. The steering line is operated by the steering mechanism to drive the connection mechanism rotate relative to the fixing member, achieving an adjustment of a circumferential position of the connection mechanism and components associated with the connection mechanism.

According to an embodiment, the shape of the fixing member is selected from one of an umbrella cover, a cylinder, a frustum, a prism, or a sleeve.

According to an embodiment, positioning of the guide channel is selected from at least one of the following: The guide channel is located in the fixing member, and the guide channel is disposed at an outer periphery of the fixing member.

According to an embodiment, the guide channel is extending in an axial direction and is disposed in the fixing member, and the guide channel is a through-hole, channel, slot, or rail.

According to an embodiment, the fixing member includes an inner sleeve and an outer sleeve sleeved on an outer periphery of the inner sleeve. The guide channel is disposed in at least one of the inner sleeve or the outer sleeve and extends substantially axially. Alternatively, the guide channel is formed by a gap between the inner sleeve and the outer sleeve.

According to an embodiment, the at least one steering line includes a first steering line and a second steering line.

According to an embodiment, the first steering line and the second steering line share one guide channel. Alternatively, each of the first steering line and the second steering line is arranged to pass through one of two guide channels independent of each other.

According to an embodiment, one of the following is disposed on an outer periphery of the connection mechanism: a first connection portion and a second connection portion that are circumferentially arranged and spaced apart, and a single connection portion protruding outwardly from the outer periphery of the connection mechanism.

According to an embodiment, the first connection portion and the second connection portion are protruding outwardly from the outer periphery of the connection mechanism and are small pillars, stubs, hooks, or bosses. One end of the first steering line and one end of the second steering line are connected to the corresponding first connection portion and the second connection portion respectively in a fastening, tethering, or binding manner. The first connection portion and the second connection portion are symmetrically disposed on two sides of the guide channel.

According to an embodiment, the steering mechanism is provided with a distaff. The first steering line passes through the guide channel and winds around one side of the distaff in a clockwise or counterclockwise direction. The second steering line passes through the guide channel and winds around another side of the distaff in a counterclockwise or clockwise direction opposite to the winding direction of the first steering line.

According to an embodiment, a first winding portion in a form of a perforation is disposed on the one side of the distaff, and a second winding portion in a form of a perforation is disposed on the another side of the distaff.

According to an embodiment, the steering mechanism further includes a hollow outer housing and a control knob for rotating the distaff. One end of the distaff is operatively connected to the control knob. Another end of the distaff is located in a hollow interior of the outer housing.

According to an embodiment, when the steering mechanism is operated to place the first steering line in a tensioned state, the second steering line is in a relaxed state; when the steering mechanism is operated to place the second steering line in a tensioned state, the first steering line is in a relaxed state.

According to an embodiment, a variable range of a rotatably adjusted angle of the connection portion is preset at -60° to 60 °.

According to an embodiment, on a cross section perpendicular to an axial direction of the connection mechanism, an included angle formed by two connection lines is α, where the two connection lines are formed by the connection of respective centers of the first connection portion and the second connection portion to a center of the connection mechanism. A variable range of the included angle α is determined by the variable range of the rotatably adjusted angle of the connection portion. An included angle formed between the steering line in a tensioned state from the connection portion to the corresponding guide channel and center lines of respective guide channels is β. The included angle α varies with the included angle β.

According to an embodiment, the included angle α satisfies: 0° < α ≤ 180°, for example, 120° ≤ α ≤ 150°.

According to an embodiment, the included angle β satisfies: 0° < β < 90°, for example, 30° < β < 75°.

According to an embodiment, based on a positioning device of any one of the preceding claims, the connection mechanism includes a connection device, a rotation device, and a limit wire. The connection device has a substantially cylindrical body. The rotation device is mounted at a proximal end of the connection device. One end of the limit wire is fixed to the rotation device. Another end of the limit wire extends through the connection device to be detachably connected to an implanted prosthesis located at a distal end of the connection device.

According to an embodiment, the implanted prosthesis is an artificial heart valve prosthesis that includes a stent body and a positioning member assembled together.

According to an embodiment, the artificial heart valve prosthesis is aortic valve prosthesis, and the number of the positioning members is three.

According to an embodiment, the positioning device further includes an inner core tube and a middle sheath tube. The connection device is sleeved on an outer periphery of the inner core tube. The rotation device is sleeved on an outer periphery of the middle sheath tube. The middle sheath tube is configured to move axially relative to the inner core tube.

According to another aspect of the present application, a rotationally adjustable positioning device is also provided, including a connection mechanism, a fixing member, and a steering line. The fixing member is provided with a guide channel. One end of the steering line is connected to the connection mechanism, and another end passes through a guide channel. The steering line is operated to drive the connection mechanism rotate circumferentially relative to the fixing member.

According to an embodiment, two guide channels are provided. The two guide channels are independent of each other to avoid the entanglement of the steering line.

According to an embodiment, part of the guide channel is located in the fixing member, while part of the guide channel is disposed at an outer periphery of the fixing member.

According to an embodiment, the guide channel may be a through hole axially disposed on the fixing member.

According to an embodiment, the through hole is configured as a rounded structure to prevent the steering line from being cut.

According to an embodiment, a guide rail is disposed on the outer periphery of the fixing member. The guide channel is disposed in the guide rail.

According to an embodiment, when the positioning device is used for aortic replacement surgery, the artificial heart valve prosthesis includes a stent body and a positioning member. The artificial heart valve prosthesis first releases the positioning member so that the positioning member is positioned at the floor of the aortic sinus to complete pre-positioning, and then the stent body is released.

According to an embodiment, the implanted prosthesis is a vascular stent or a valve clip.

According to an embodiment, when the implanted prosthesis needs to be adjusted in the circumferential direction when transported into the human body, the steering mechanism may be operated so that the steering line drives the connection mechanism and further drives the implanted prosthesis to rotate in the circumferential direction.

According to an embodiment, the positioning device includes a limit wire, the connection device includes a connection hole, and the implanted prosthesis is provided with a disassembly hole. During pre-installation, the disassembly hole passes through the connection hole. One end of the limit wire passes through the disassembly hole to complete the connection. Another end of the limit wire extends for extracorporeal operation.

According to an embodiment, the steering line is selected from one of the following: a thread, a rope, a cable, a strand, a twine, a wire, a flexible belt, and any combination of the preceding material.

According to another aspect of the present application, a rotationally adjustable positioning device is also provided, including a fixing member, a connection mechanism, a first steering line, and a second steering line. The connection mechanism is substantially extending in a longitudinal direction and is rotatably connected to a distal side of the fixing member. A first connection portion and a second connection portion that are circumferentially arranged and spaced apart are disposed on an outer periphery of the connection mechanism. The fixing member is provided with a guide channel. The first steering line and the second steering line share one guide channel, or each of the first steering line and the second steering line is arranged to pass through one of two guide channels independent of each other. One end of the first steering line and one end of the second steering line are connected to the first connection portion and the second connection portion respectively. Another end of the first steering line and another end of the second steering line are connected to a corresponding part of the steering mechanism through a corresponding guide channel. When the steering mechanism is operated to place the first steering line in a tensioned state, the second steering line is in a relaxed state; when the steering mechanism is operated to place the second steering line in a tensioned state, the first steering line is in a relaxed state. In this manner, the connection mechanism is made to be rotatable in two opposite rotational directions relative to the fixing member so that a circumferential position of the connection mechanism and components associated with the connection mechanism can be adjusted.

According to another aspect of the present application, a rotationally adjustable positioning device is also provided, including a fixing member, a connection mechanism, and a single steering line. The connection mechanism is substantially extending in a longitudinal direction and is rotatably connected to a distal side of the fixing member. A single connection point is disposed on the connection mechanism. The fixing member is provided with a single guide channel. One end of the steering line is connected to an elastic reset mechanism wound around the single connection point of the connection mechanism, and another end is connected to the steering mechanism through the guide channel. The steering mechanism is operated to place the steering line in a tensioned state to drive the connection mechanism rotate relative to the fixing member. After the steering line is loosened, the elastic reset mechanism causes the connection mechanism to reversely rotate.

Compared with the existing art, the technical solution of the present application has at least the following advantages:
In the existing art, the end of the tube is usually controlled and rotated so that the force is transmitted to the other end of the tube through the rotation to achieve the purpose of adjusting the angle. However, the tube generates a torsional force during rotation, resulting in inaccurate adjustment. In particular, when the tube has a longer length longer or in the face of a complex twisted blood vessel shape, the accuracy of adjustment is drastically reduced, and problems of adjustment delay or even inability to adjust exist. When the tube is adjusted in the vascular approach, because the blood vessel has multiple twists to a certain extent, the difficulty and timeliness of the adjustment are further increased and affected, making it difficult for the positioning of the procedure. According to a concept of the present application, the angle formed between the connection end and the guide channel on the cross section of the fixing member can be adjusted by a pull of the steering line. Most of the pull force is concentrated between the guide channel and the connection end. The loss is extremely small, and feedback on the adjustment is very timely and accurate.

According to a concept of the present application, the steering line includes a first steering line and a second steering line. By clockwise manipulation of the control mechanism, the first steering line can cause the connection mechanism to rotate in the clockwise direction. By counterclockwise manipulation of the control mechanism, the second steering line can cause the connection mechanism to rotate in the counterclockwise direction. Based on the concept, the structure is simple, the adjustment is very convenient, and the assembly requirement for the positioning device is low.

According to a concept of the present application, one end of the first steering line is connected to a first winding portion, and another end of the first steering line is partially wound on the distaff in a clockwise direction. One end of the second steering line is connected to the second winding portion, and another end of the second steering line is partially wound on the distaff in a counterclockwise direction. The configuration of the concept can satisfy the adjustment of the implanted prosthesis in the circumferential direction and the angle. Moreover, the design and assembly of the concept are simple, and the cost is low. In this manner, it is more favorable for mass industrial production with low cost and high reliability.

According to the preceding and other concepts of the technical solution of the present application, many technical problems and deficiencies present in the existing art can be solved. For example, the circumferential position of the valve prosthesis can no longer be accurately adjusted after the positioning member is released so that the positioning of the valve prosthesis into the sinus is not sufficiently accurate. Unlimited release of the valve prosthesis may lead to deviation or even disengagement of the stent from the intended position when the stent is released in the heart.

Embodiments of the present application are capable of achieving other advantageous technical effects not listed one by one. These other technical effects may be partially described below and are expected and understood by those skilled in the art upon reading the present application.

### BRIEF DESCRIPTION OF DRAWINGS

With reference to the following description in conjunction with drawings, the preceding and other features and advantages of these embodiments, and how these embodiments are implemented, become more apparent. Moreover, a better understanding of embodiments of the present application can be obtained. In drawings:
FIG. 1a is a view of a connection mechanism, a fixing member, and a steering line according to an embodiment of the present application.
FIG. 1b is another view of the embodiment shown in FIG. 1 after rotation by a certain angle in the circumferential direction.
FIGS. 2a to 2d are diagrams of different forms of guide channels and diagrams illustrating the relationship among the guide channel, steering line and connection portion.
FIGS. 3a and 3b are diagrams of an implanted prosthesis positioning device according to an embodiment of the present application.
FIGS. 4a to 4d are diagrams illustrating the structure of a steering mechanism according to an example of the present application.
FIGS. 5a to 5g are diagrams of an implanted prosthesis positioning device according to another embodiment of the present application.
FIGS. 6a to 6c are diagrams of another example of a fixing member according to the present application.
FIGS. 7a to 7c are diagrams illustrating the structure of another embodiment according to the present application.
FIGS. 8a to 8c are diagrams illustrating the structure of another embodiment according to the present application.

Each number in the drawings refers to the feature described below:
- 1: connection mechanism
- 11: first connection portion
- 12: second connection portion
- 13: connection device
- 131: through hole
- 14: rotation device
- 15: limit wire
- 2: fixing member
- 21: guide channel
- 22: inner sleeve
- 23: outer sleeve
- 3: steering line
- 31: first steering line
- 32: second steering line
- 33: elastic reset mechanism
- 4: implanted prosthesis
- 41: stent body
- 42: positioning member
- 5: control mechanism
- 51: outer housing
- 52: distaff
- 521: first winding portion
- 522: second winding portion
- 53: control knob
- 6: inner core tube
- 7: middle sheath tube

### DETAILED DESCRIPTION

Details of one or more embodiments of the present application are set forth in the description of drawings and specific implementations below. Other features, objects, and advantages of the present application are apparent from the description, drawings, and claims.

It should be understood that the illustrated and described embodiments are not limited, in application, to the details of construction and arrangement of components set forth in the description described below or illustrated in the drawings. The illustrated embodiments may be other embodiments and can be practiced or carried out in various ways. Each example is provided by way of explanation, not limitation, of the disclosed embodiments. In fact, it is apparent to those skilled in the art that various modifications and variations may be made in various embodiments of the present application without departing from the scope or spirit of the present application. For example, features illustrated or described as part of one embodiment, may be used with another embodiment to still yield a further embodiment. Accordingly, it is intended that the present application covers such modifications and variations as come within the scope of the appended claims and equivalents thereof.

Also, it is to be understood that the phrases and terms used herein are for the purpose of description and should not be considered as limiting. The use of "including", "comprising", or "having" and variations thereof herein is intended to openly include the items listed thereafter and equivalents thereof, as well as additional items.

The present application is described in more detail below with reference to different embodiments and examples of several aspects of the present application. In the present application, the term "proximal end" or "proximal side" refers to the end or side closer to the operator, and "distal end" or "distal side" refers to the end or side farther away from the operator.

In the existing art, the end of the tube is usually controlled and rotated so that the force is transmitted to the other end of the tube through the rotation to achieve the purpose of adjusting the angle. However, the tube generates a torsional force during rotation, resulting in inaccurate adjustment. In particular, when the tube has a longer length longer or in the face of a complex twisted blood vessel shape, the accuracy of adjustment is drastically reduced, and problems of adjustment delay or even inability to adjust exist. When the tube is adjusted in the vascular approach, because the blood vessel has multiple twists to a certain extent, the difficulty and timeliness of the adjustment are further increased and affected, making it difficult for the positioning of the operation.

One of the objectives of the embodiments described below is to address the preceding deficiencies, as well as other problems.

### Embodiment one

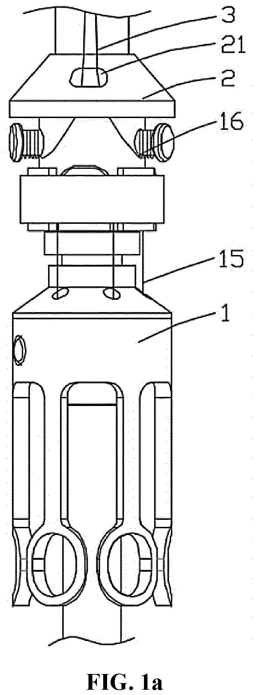

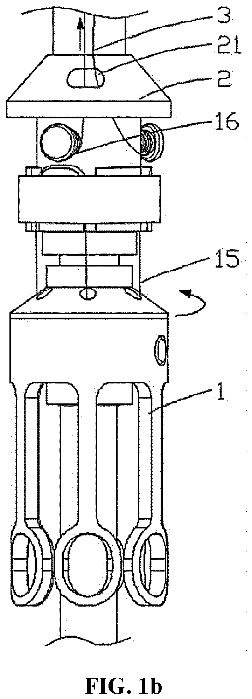

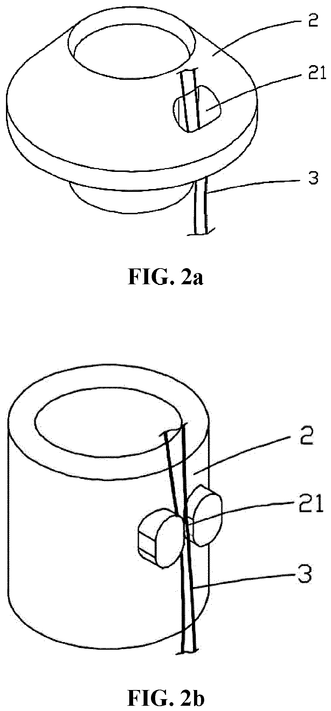

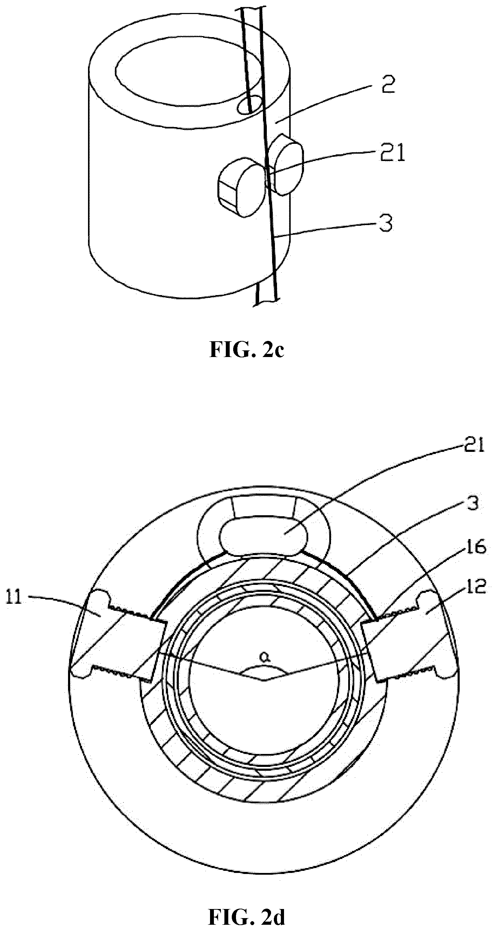

As shown in FIGS. 1a and 1b, a rotatably adjusted positioning device for aortic valve surgery is illustrated according to an embodiment of the present application. The device includes a fixing member 2, a connection mechanism 1, and a steering line 3. The connection mechanism 1 is substantially extending in a longitudinal direction and is rotatably connected to the distal side of the fixing member 2. The steering line 3 is in a flexible form such as a thread, a rope, a cable, a strand, a twine, a wire, or a flexible belt. The fixing member 2, which may have a substantially umbrella cover shape, is provided with a guide channel 21 in the form of, for example, a through hole or a channel, as shown in FIG. 2a. As shown in FIGS. 1a and 1b, one end of the steering line 3 is connected to the connection mechanism 1, for example, is fastened, tethered or wound at the connection end 16 of the connection mechanism 1, and the other end of the steering line 3 extends through the guide channel 21 of the fixing member 2 to be operatively connected to a steering mechanism 5. A connection portion may be disposed at the connection end 16. For example, the connection portion may be a radially protruding small pillar or stub for the convenience of, for example, fastening, tethering, or winding the steering line 3. FIGS. 1a and 1b show two connection portions in the form of small pillars or stubs arranged circumferentially along the body of the connection mechanism 1, which is described in detail below. In this manner, for example, by the pull and manipulation of the steering line 3, the connection mechanism 1 can be moved circumferentially relative to the fixing member 2, for example, can be rotated. In this embodiment, by the pull and manipulation of the steering line 3, the included angle α formed between the connection end 16 and the guide channel 21 on the cross section perpendicular to the axial direction of the connection mechanism 1 can be adjusted (as shown in FIG. 2d). Most of the pulling force is concentrated on the steering line 3 between the guide channel 21 and connection end 16. In this manner, the loss to the steering line 3 and the entire device is minimal, the feedback on the pulling adjustment is very timely, and the accuracy is high.

In this embodiment, as one preferred example, the guide channel 21 may be configured as a through hole or channel passing through the body of the fixing member 2 and substantially extending in the preset direction of pulling the steering line 3. As shown in FIG. 2a, for example, the guide channel extends substantially axially.

As another example, the guide channel 21 may be disposed on an outer periphery of the fixing member 2 having a substantially cylindrical body, as shown in FIG. 2b. The guide channel 21 is in the form of an axially extending channel passing between two radially protruding bosses spaced apart in the circumferential direction of the substantially cylindrical body of the fixing member 2.

As another example, the guide channel 21 is in the form of a combination of the guide channel shown in FIG. 2a and the guide channel shown in FIG. 2b. Each of the guide channels accommodates a portion of the steering line 3 so that the steering line 3 is guided and pulled during operation. In this example, the number of guide channels 21 may be two. The two guide channels 21 are independent of each other to avoid entanglement of the steering line 3 during operation.

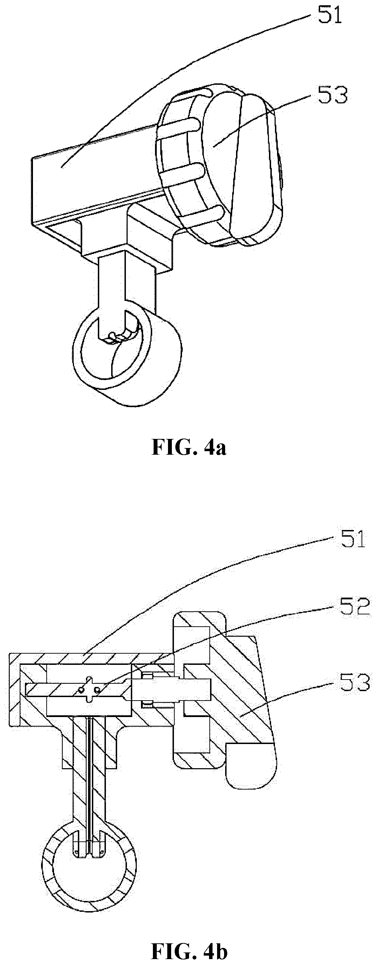

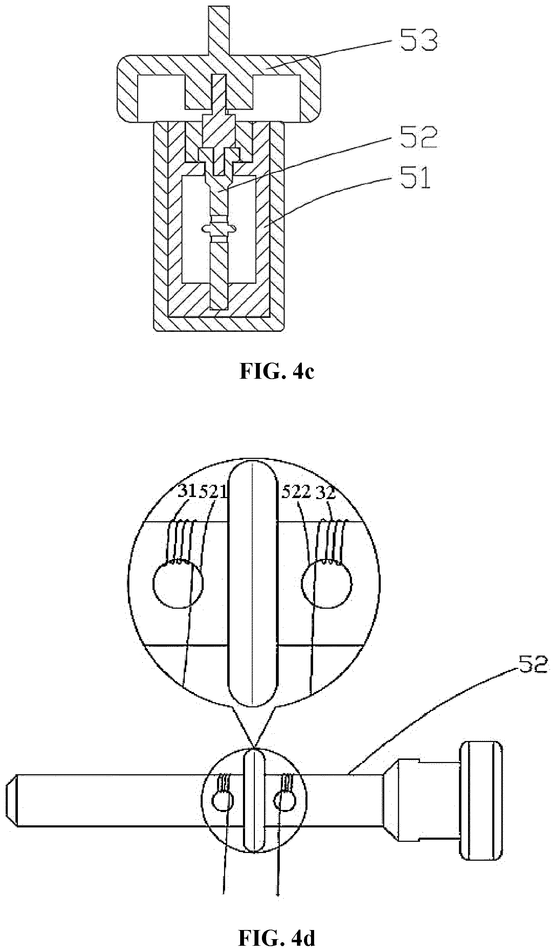

In this embodiment, the positioning device may also include an implanted prosthesis 4 and a steering mechanism 5. The implanted prosthesis 4 may be connected to the distal end or side of the connection mechanism 1 and may be rotated with the connection mechanism 1. The other end of the steering line 3, namely, the end designed to be connected to the steering mechanism 5, extends through the guide channel 21 and is connected to the steering mechanism 5 (for example, as shown in FIGS. 4a to 4d). Thus, an operator, such as a surgeon, can manipulate the steering mechanism 5 to facilitate winding (tensioning) and releasing (relaxing) of the steering line 3, thereby manipulating the connection mechanism 1 connected with the steering line 3 to rotate circumferentially relative to the fixing member 1.

According to an example, the steering mechanism 5 may include an outer housing 51, a distaff 52, and a control knob 53 assembled together, as shown in FIGS. 4a to 4D. One end of the distaff 52 is fixedly connected to the control knob 53. Another end of the distaff 52 protrudes and is disposed inside the hollow outer housing 51. The steering line 3 may be wound on the distaff 52. For example, a first winding portion 521 in the form of a perforation and a second winding portion 522 in the form of a perforation are disposed on the distaff 522, as shown in FIG. 4d. The first steering line 31 is fixed to the first winding portion 521 in the form of, for example, fastening, binding, or adhesion and is wound on the distaff 52 in a clockwise or counterclockwise direction (for example, the first winding portion 521 may be wound for several turns). The second steering line 32 is likewise fixed to the second winding portion 522 in the form of, for example, fastening, binding, or adhesion and is wound on the distaff 52 in a direction opposite to the winding direction of the first steering line 31, that is, in a counterclockwise or clockwise direction (for example, the second winding portion 522 may be wound for several turns).

When the control knob 53 is rotated clockwise, the first steering line 31 is in a tensioned (wound) state and further drives the connection mechanism 1 connected to the first steering line 31, for example, to rotate clockwise. At this time, the second steering line 32 is in a relaxed (released) state.

When the control knob 53 is rotated counterclockwise, the second steering line 32 is in a tensioned (wound) state and further drives the connection mechanism 1 connected to the second steering line 32, for example, to rotate counterclockwise. At this time, the first steering line 31 is in a relaxed (released) state.

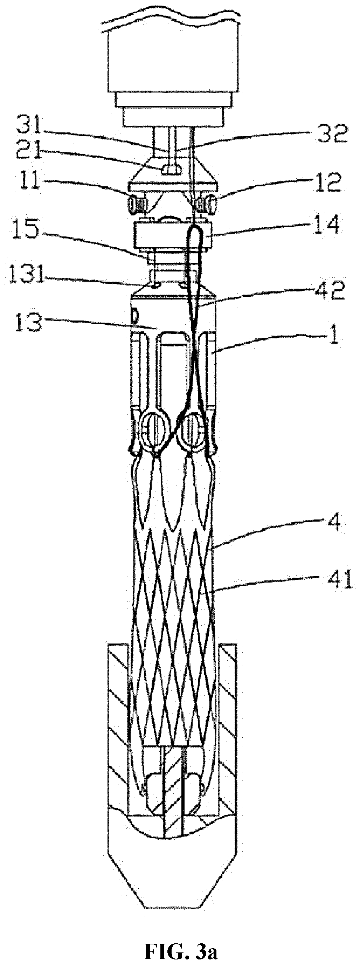

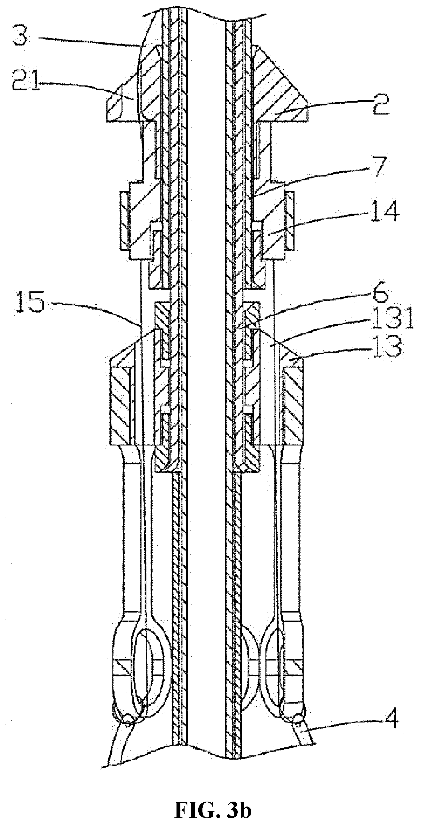

According to one example, the connection mechanism 1 may include a connection device 13, a rotation device 14, and a limit wire 15. The connection device 13 may have a substantially cylindrical body. The rotation device 14 is mounted at the proximal end of the connection device 13. One end of the limit wire 15 is fixed to the rotation device 14, for example, is fastened or tethered on the rotation device 14. Another end of the limit wire passes through the through hole 131 on the connection device 13 to be detachably connected to an implanted prosthesis 4 located at a distal end of the connection device 13, as shown in FIGS. 3a and 3b.

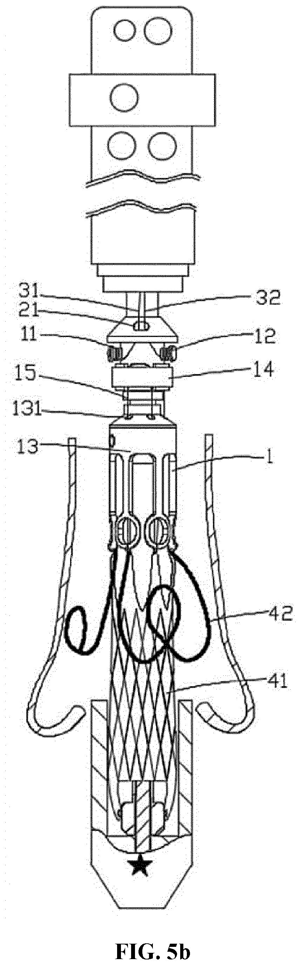

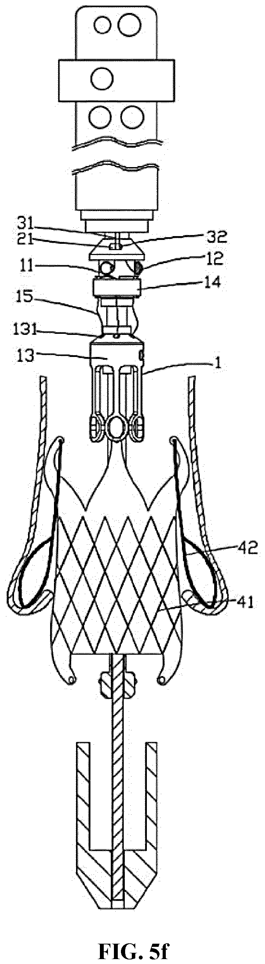

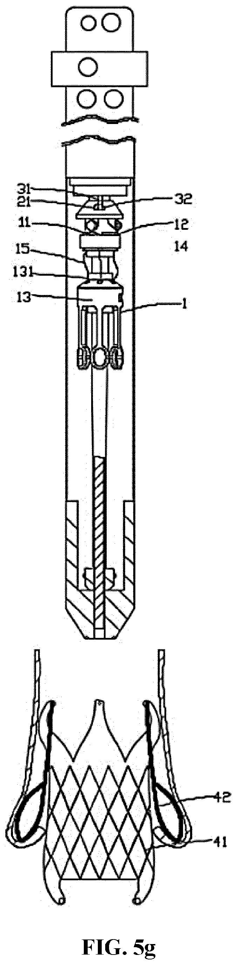

The implanted prosthesis 4 may be an artificial heart valve prosthesis that includes a stent body 41 and a positioning member 42 assembled together, as shown in FIGS. 3a and 5b.

According to an example, the pre-positioning of the artificial heart valve prosthesis may be accomplished by the first release of the positioning member 42 to be positioned to the floor of the aortic sinus, and then the stent body 41 is released in a subsequent surgical procedure.

In the surgical procedure, when circumferential adjustment of the angle is required during the delivery of the implanted prosthesis 4 into the heart of the human body, the operator can operate the control knob 53 of the steering mechanism 5 to drive the steering line 3 wound around the distaff 52, as shown in FIGS. 4a to 4d, so that the steering line 3 drives the connection mechanism 1 connected to the steering line 3 to rotate clockwise or counterclockwise relative to the fixing member 2. This rotation further drives the implanted prosthesis 4 connected to the connection mechanism 1 to rotate circumferentially, as described in detail below.

In the surgical procedure, a first connection portion 11 and a second connection portion 12, for example, in the form of small pillars or stubs, are disposed at the connection end 16 of the connection mechanism 1, as shown in FIG. 3a. The steering line 3 is connected to the first connection portion 11 and the second connection portion 12 in the manner of, for example, fastening or tethering. When the first steering line 31 pulls the first connection portion 11 to rotate circumferentially, the implanted prosthesis 4 connected to the connection mechanism 1 can, for example, be rotated circumferentially in a clockwise direction. When the second steering line 32 pulls the second connection portion 12 to rotate circumferentially, the implanted prosthesis 4 connected to the connection mechanism 1 can, for example, be rotated circumferentially in a counterclockwise direction.

On a cross section perpendicular to the axial direction of the connection mechanism, an included angle formed by two connection lines is α, where the two connection lines are formed by connection of respective centers of the first connection portion 11 and the second connection portion 12 to a center of the connection mechanism 1. The variable range of the included angle α is determined by the variable range of the rotatably adjusted angle of the connection portion 11.

According to an example, the included angle α satisfies: 0° < α ≤ 180°, for example, 120° ≤ α ≤ 150°.

Since the aortic sinus has 3 sinus floors that are evenly distributed on the circumference, the angle formed by lines between the adjacent sinus floors and the center of the aortic valve is about 120°. Three positioning members 42 of the implanted prosthesis 4 are provided to match the number of sinus floors of the aortic sinus. As described above, the surgeon can conveniently and accurately drive the implanted prosthesis 4 to rotate clockwise or counterclockwise by manipulating the steering mechanism 5. In this manner, for example, the positioning member 42 is positioned to the corresponding sinus floor. When delivered into the heart, the positioning member 42 has the maximum angle of rotation of about 60°in the counterclockwise or clockwise direction. Therefore, a preferred value of the included angle α is about 120°.

According to an example, viewed on the cross section of the fixing member 2 perpendicular to the axial direction, the first connection portion 11 and the second connection portion 12 are symmetrically disposed left and right with respect to the center of the guide channel 21. With this arrangement, it can be ensured that the implanted prosthesis 4 can be conveniently and precisely adjusted to an ideal implantation position as desired in the circumferential direction, no matter in the counterclockwise direction or in the clockwise direction.

According to an example, the positioning device further includes an inner core tube 6 and a middle sheath tube 7, as shown in FIG. 3b. The connection device 13 is sleeved on an outer periphery of the inner core tube 6. The rotation device 14 is sleeved on an outer periphery of the middle sheath tube 7. The middle sheath tube 7 is configured to move axially relative to the inner core tube 6. The middle sheath tube 7 moves axially towards the proximal end relative to the inner core tube 6 so that assembly and disassembly of the connection device 13 and the implanted prosthesis 4 can be achieved.

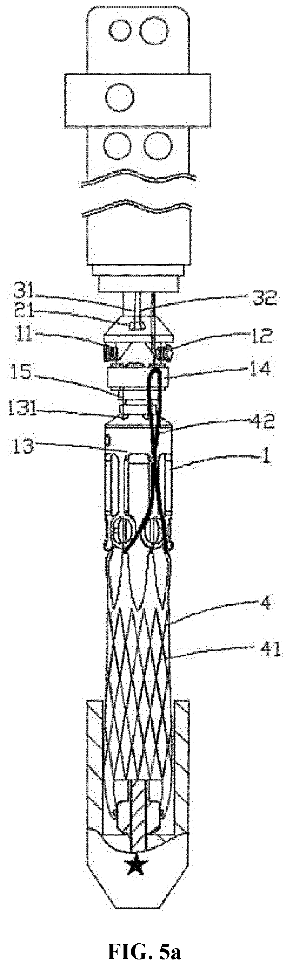

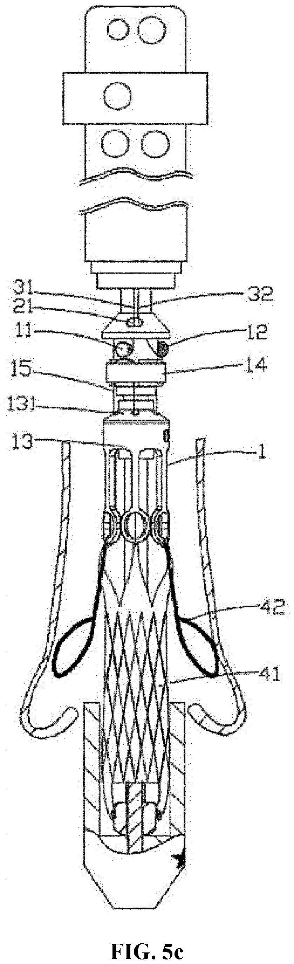

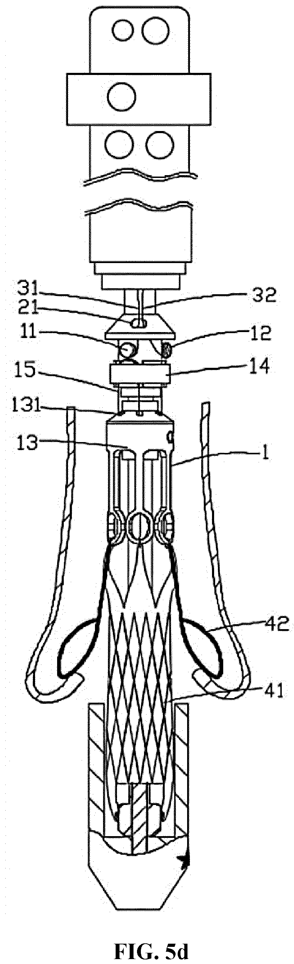

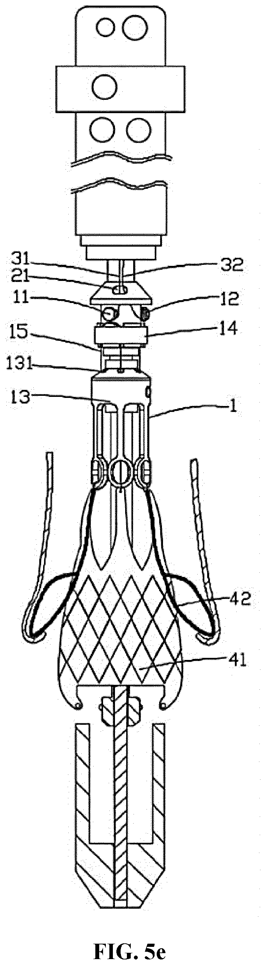

The operation of the positioning device includes the following steps:
(1) The positioning device is operated to enter the heart through the transvascular approach, and the positioning member 42 on the implanted prosthesis 4 is then released to the desired position in the heart, as shown in FIGS. 5a and 5b.
(2) The current position of the positioning member 42 with respect to the aortic sinus is observed. If the position does not reach the ideal expected position, the surgeon can pull the corresponding steering line 3 by operating the steering mechanism 5 to make the steering line 3 drive the rotation of the rotation device 14 of the connection mechanism 1 connected to the steering line 3. The rotation of the rotation device 14 in turn drives the rotation of the limit wire 15 connected to the rotation device 14. The limit wire 15 in turn drives the connection device 13 connected to the limit wire 15 and the implanted prosthesis 4 to rotate together to achieve the adjustment of the position of the positioning member 42 of the implanted prosthesis 4. In this manner, the positioning member 42 is finally positioned to the ideal expected position of the sinus floor, as shown in FIG. 5c.
(3) When the positioning member 42 reaches the ideal expected position of the sinus floor, as shown in FIG. 5d, the stent body 41 is further released in place. Afterwards, the positioning device is withdrawn to complete the implantation procedure, as shown in FIGS. 5e to 5g.

### Embodiment two

Embodiment two is substantially the same as embodiment one, except that the fixing member has a different configuration.

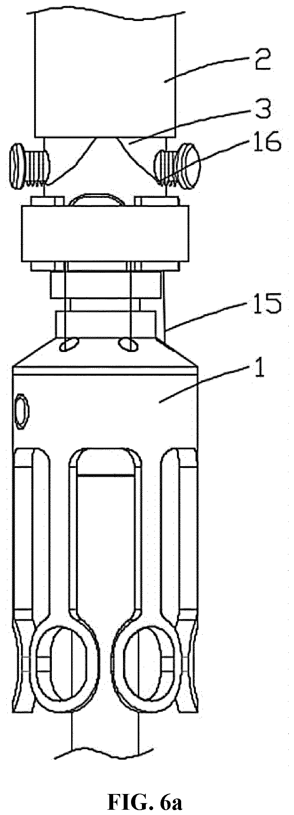

As shown in FIG. 6a, a rotatably adjusted positioning device for aortic valve surgery is illustrated. The device includes a fixing member 2, a connection mechanism 1, and a steering line 3. The connection mechanism 1 is substantially extending in a longitudinal direction and is rotatably connected to the distal side of the fixing member 2. The steering line 3 is in a flexible form such as a thread, a rope, a cable, a strand, or a wire.

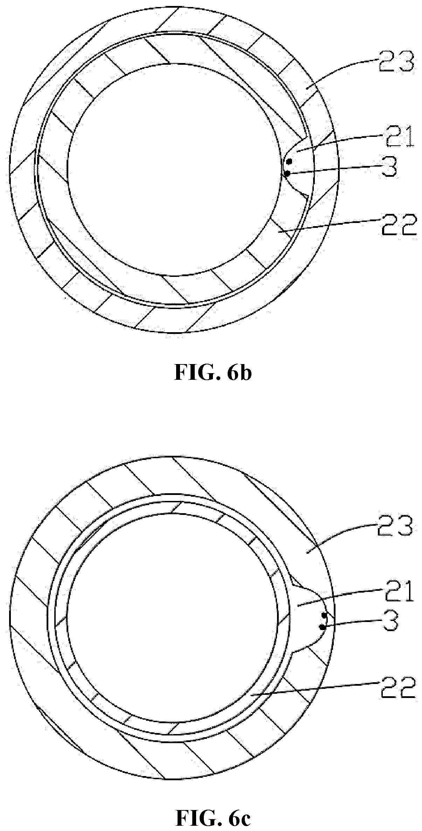

In embodiment two, the fixing member 2 includes an inner sleeve 22 and an outer sleeve 23 sleeved on an outer periphery of the inner sleeve 22. The guide channel 21 substantially axially extending is disposed in the fixing member 2, and the guide channel is in the form of a through-hole, a channel, or a slot. As shown in FIG. 6b, the guide channel 21 may be an axially extending slot disposed at the inner sleeve 22 and between the inner sleeve 22 and the outer sleeve 23 so as to facilitate the passage of the steering line 3. As shown in FIG. 6c, the guide channel 21 may be an axially extending slot disposed at the outer sleeve 23 and between the inner sleeve 22 and the outer sleeve 23 so as to facilitate the passage of the steering line 3.

Generally similar to embodiment one, one end of the steering line 3 is connected to the connection mechanism 1, for example, is fastened, tethered or wound at the connection end 16 of the connection mechanism 1. The other end of the steering line 3 extends through the guide channel 21 of the fixing member 2 to be operatively connected to a steering mechanism 5. In this manner, the steering line 3 can be pulled and manipulated by manipulation of the steering mechanism 5 so that the rotation device 14 of the connection mechanism 1 connected to the steering line 3 can rotate in the circumferential direction relative to the fixing member 2. The rotation device 14 in turn drives the rotation of the limit wire 15 connected to the rotation device 14. The limit wire 15 in turn drives the connection device 13 connected to the limit wire 15 and the implanted prosthesis 4 to rotate together to achieve the adjustment of the position of the positioning member 42 of the implanted prosthesis 4. In this manner, the positioning member 42 is finally positioned to the ideal expected position of the sinus floor.

In this regard, the relevant construction and concept of embodiment two are similar to those of embodiment one, and therefore the description is not repeated herein.

### Embodiment three

Embodiment three is substantially the same as embodiment one, except that embodiment three employs a single connection portion 11, a single steering line 3, and two guide channels 21.

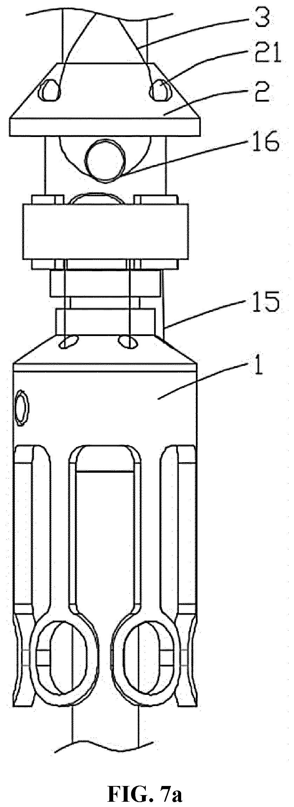

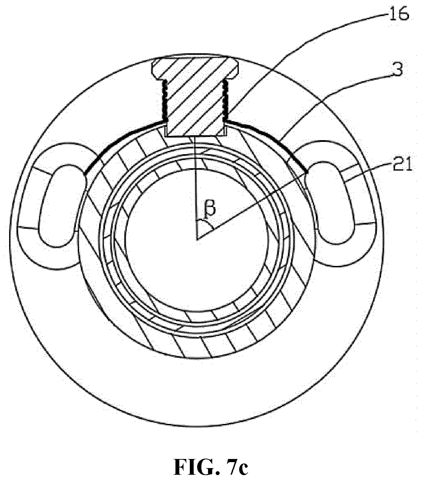

As shown in FIG. 7a, a rotatably adjusted positioning device for aortic valve surgery is illustrated. The device includes a fixing member 2, a connection mechanism 1, and a steering line 3. The connection mechanism 1 is substantially extending in a longitudinal direction and is rotatably connected to the distal side of the fixing member 2. The steering line 3 is in a flexible form such as a thread, a rope, a cable, a strand, or a wire.

In this embodiment three, the guide channel 21 may be configured as a through hole or channel passing through the body of the fixing member 2 and substantially extending in the preset direction of pulling the steering line 3. As shown in FIG. 7a, for example, the guide channel extends substantially axially.

In this embodiment 3, the number of guide channels 21 may be two. The two guide channel 21 are independent of each other to avoid entanglement of the steering line 3 during operation (in this embodiment, a preset angle exists between the two guide channels).

In this embodiment three, the variable range of a rotatably adjusted angle of the connection portion 11 is preset at -60° to 60°.

In this embodiment three, an included angle formed between the steering line 3 in a tensioned state from the connection portion 11 to the corresponding guide channel 21 and center lines of respective guide channels is β. The included angle β satisfies: 0° < β < 90°, for example, 30° < β < 75°.

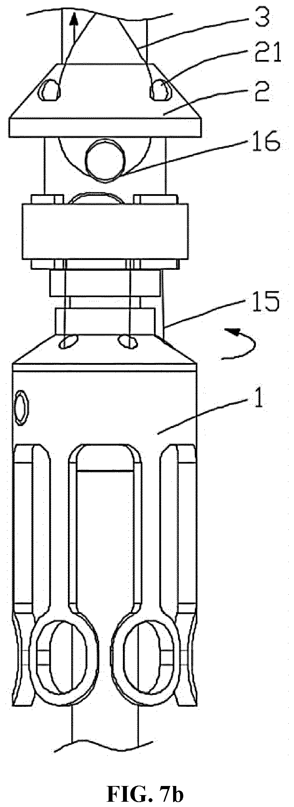

In this embodiment 3, as shown in FIG. 7b, the steering line 3 is one line, and the steering line 3 is cooperatively connected to the connection portion 11. The connection manner may be fastening, tethering, binding, or other fixed mode. One end of the steering line 3 passes through the first guide channel 211. The other end of the steering line 3 passes through the second guide channel 212. Moreover, two ends of the steering line 3 are operatively connected to the steering mechanism 5. In this manner, the steering line 3 can be pulled and manipulated by manipulation of the steering mechanism 5 so that the rotation device 14 of the connection mechanism 1 connected to the steering line 3 can rotate in the circumferential direction relative to the fixing member 2. The rotation device 14 in turn drives the rotation of the limit wire 15 connected to the rotation device 14. The limit wire 15 in turn drives the connection device 13 connected to the limit wire 15 and the implanted prosthesis 4 to rotate together to achieve the adjustment of the position of the positioning member 42 of the implanted prosthesis 4. In this manner, the positioning member 42 is finally positioned to the ideal expected position of the sinus floor.

In this regard, the relevant construction and concept of embodiment three are similar to those of embodiment one, and therefore the description is not repeated herein.

### Embodiment four

Embodiment four is substantially the same as embodiment one, except that embodiment four employs a single connection portion 11, a single guide channel 21, and a single steering line 3, and that the steering line includes an elastic reset mechanism.

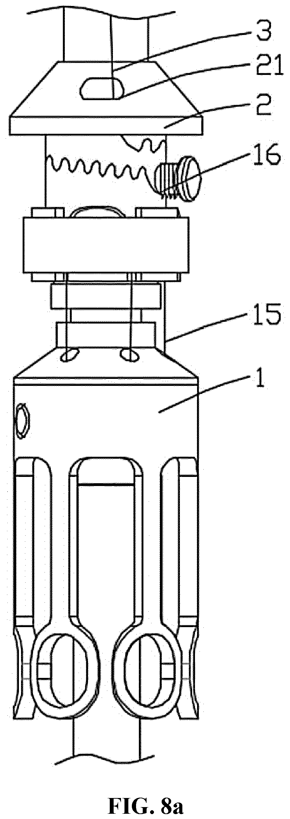

As shown in FIG. 8a, a rotatably adjusted positioning device for aortic valve surgery is illustrated.

The device includes a fixing member 2, a connection mechanism 1, and a single steering line 3. The connection mechanism 1 is substantially extending in a longitudinal direction and is rotatably connected to a distal side of the fixing member 2. The single connection portion 11 is disposed on the connection mechanism 1. The fixing member 2 is provided with a single guide channel 21. One end of the steering line 3 is connected to an elastic reset mechanism 33 wound around the single connection portion 11, and another end is connected to the steering mechanism 5 through the guide channel 21. The steering mechanism 5 is operated to place the steering line 3 in a tensioned state to make the connection mechanism 1 rotate relative to the fixing member 2. After the steering line 3 is loosened, the elastic reset mechanism 33 causes the connection mechanism 1 to reversely rotate.

In this embodiment four, the elastic reset mechanism 33 may be configured as a spring or other components with an elastic reset function.

In this embodiment four, the guide channel 21 may be configured as a through hole or channel passing through the body of the fixing member 2 and substantially extending in the preset direction of pulling the steering line 3. As shown in FIG. 7a, for example, the guide channel extends substantially axially.

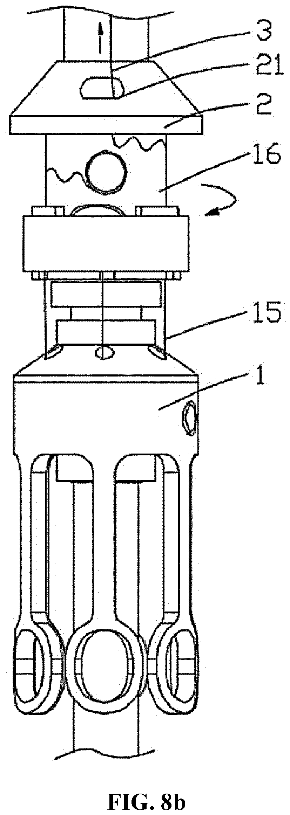

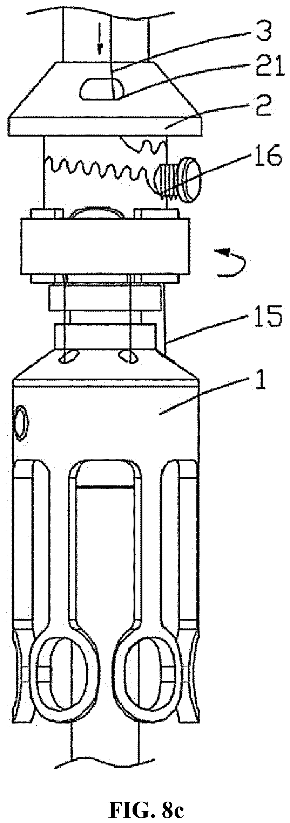

In this embodiment four, as shown in FIGS. 8b and 8c, the steering line 3 is one line, and the steering line 3 is cooperatively connected to the connection portion 11. The connection manner may be fastening, tethering, binding, or other fixed mode. One end of the steering line 3 is connected to the connection portion 11. The elastic reset mechanism 33 of the steering line 3 is wound around the connection mechanism 1. The other end of the steering line 3 extends through the guide channel 21 to be operatively connected to a steering mechanism 5. In this manner, the steering line 3 can be pulled and manipulated by manipulation of the steering mechanism 5 so that the rotation device 14 of the connection mechanism 1 connected to the steering line 3 can rotate in the circumferential direction relative to the fixing member 2. The rotation device 14 in turn drives the rotation of the limit wire 15 connected to the rotation device 14. The limit wire 15 in turn drives the connection device 13 connected to the limit wire 15 and the implanted prosthesis 4 to rotate together to achieve the adjustment of the position of the positioning member 42 of the implanted prosthesis 4. Moreover, when the rotation angle needs to be adjusted in the opposite direction, it is only necessary to loosen the steering line 3. With an elastic restoring force, the elastic reset mechanism 33 of the steering line 3 can be rotated in the opposite direction to adjust a certain angle. In this manner, the positioning member 42 is finally positioned to the ideal expected position of the sinus floor.

In this regard, the relevant construction and concept of embodiment four are similar to those of embodiment one, and therefore the description is not repeated herein.

The foregoing description of several embodiments for the present application is presented for illustration. The foregoing description is not intended to be exhaustive or to limit the present application to the precise configurations, constructions, and/or steps disclosed. Obviously, many modifications and variations are possible in light of the preceding teachings. The scope of the present invention and all equivalents are intended to be defined by the appended claims.

## Claims

1. A rotatably adjusted positioning device, comprising:
a fixing member,
a connection mechanism substantially extending in a longitudinal direction and rotatably connected to a distal side of the fixing member, and
at least one steering line,
wherein the fixing member is provided with at least one guide channel,
an end of the steering line is connected to the connection mechanism, and another end of the steering line is connected to a steering mechanism through a respective one of the at least one guide channel,
wherein the steering line is operated by the steering mechanism to drive the connection mechanism rotate relative to the fixing member, to adjust a circumferential position of the connection mechanism and components associated with the connection mechanism.

2. The positioning device according to claim 1, wherein a shape of the fixing member comprises one of an umbrella cover, a cylinder, a frustum, a prism, or a sleeve.

3. The positioning device according to claim 1, wherein positioning of the at least one guide channel comprises at least one of the following:
the at least one guide channel is located in the fixing member; and
the at least one guide channel is disposed at an outer periphery of the fixing member.

4. The positioning device according to claim 1, wherein the at least one guide channel is extending in an axial direction and is disposed in the fixing member, and the at least one guide channel is a through-hole, channel, slot, or rail.

5. The positioning device according to claim 4, wherein the fixing member comprises an inner sleeve and an outer sleeve sleeved on an outer periphery of the inner sleeve,
wherein the at least one guide channel is disposed in at least one of the inner sleeve or the outer sleeve and extends substantially axially; or, the guide channel is formed by a gap between the inner sleeve and the outer sleeve.

6. The positioning device according to any one of claims 1 to 5, wherein the at least one steering line comprises a first steering line and a second steering line.

7. The positioning device according to claim 6, wherein the first steering line and the second steering line share a same guide channel of the at least one guide channel; or, the first steering line and the second steering line are arranged to pass through two guide channels of the at least one guide channel respectively.

8. The positioning device according to any one of claims 1 to 6, wherein one of the following is disposed on an outer periphery of the connection mechanism:
a first connection portion and a second connection portion that are circumferentially arranged and spaced apart; and
a single connection portion protruding outwardly from the outer periphery of the connection mechanism.

9. The positioning device according to claim 8, wherein the first connection portion and the second connection portion are protruding outwardly from the outer periphery of the connection mechanism and are small pillars, stubs, hooks, or bosses, and an end of the first steering line and an end of the second steering line are connected to the first connection portion and the second connection portion respectively in a fastening, tethering, or binding manner,
wherein the first connection portion and the second connection portion are symmetrically disposed on two sides of the at least one guide channel.

10. The positioning device according to any one of claims 6 to 9, wherein the steering mechanism is provided with a distaff, the first steering line is configured to pass through a respective one of the at least one guide channel and winds around one side of the distaff in a clockwise or counterclockwise direction, and the second steering line is configured to pass through a respective one of the at least one guide channel and winds around another side of the distaff in a counterclockwise or clockwise direction opposite to the winding direction of the first steering line.

11. The positioning device according to claim 10, wherein a first winding portion in a form of a perforation is disposed on the side of the distaff, and a second winding portion in a form of a perforation is disposed on the another side of the distaff.

12. The positioning device according to claim 10 or 11, wherein the steering mechanism further comprises a hollow outer housing and a control knob for rotating the distaff,
wherein an end of the distaff is operatively connected to the control knob, and another end of the distaff is located in a hollow interior of the outer housing.

13. The positioning device according to any one of claims 6 to 12, wherein the first steering line is operated by the steering mechanism in a tensioned state, the second steering line is in a relaxed state; and
when the second steering line is operated by the steering mechanism in a tensioned state, the first steering line is in a relaxed state.

14. The positioning device according to any one of claims 8 to 13, wherein a variable range of a rotatably adjusted angle of the connection portion is preset at -60° to 60°.

15. The positioning device according to claim 8 to 14, wherein on a cross section perpendicular to an axial direction of the connection mechanism, an included angle formed by two connection lines is α, wherein the two connection lines are formed by connection of respective centers of the first connection portion and the second connection portion to a center of the connection mechanism, and a variable range of the included angle α is determined by the variable range of the rotatably adjusted angle of the connection portion; and
wherein an included angle formed between the steering line in a tensioned state from the connection portion to the corresponding guide channel and a center line of respective guide channel is β, wherein the included angle α varies with the included angle β.

16. The positioning device according to according to any one of the preceding claims, wherein the connection mechanism comprises:
a connection device having a substantially cylindrical body,
a rotation device mounted at a proximal end of the connection device, and
a limit wire,
wherein an end of the limit wire is fixed to the rotation device, and another end of the limit wire extends through the connection device and is detachably connected to an implanted prosthesis located at a distal end of the connection device.

17. The positioning device according to claim 16,
wherein the positioning device further comprises an inner core tube and a middle sheath tube,
wherein the connection device is sleeved on an outer periphery of the inner core tube, the rotation device is sleeved on an outer periphery of the middle sheath tube, and the middle sheath tube is configured to move axially relative to the inner core tube.

18. The positioning device of any one of the preceding claims, wherein the steering line comprises at least one of the following: a thread, a rope, a cable, a strand, a twine, a wire, or a flexible belt.

19. A rotatably adjusted positioning device, comprising:
a fixing member,
a connection mechanism substantially extending in a longitudinal direction and rotatably connected to a distal side of the fixing member, wherein a first connection portion and a second connection portion that are circumferentially arranged and spaced apart are disposed on an outer periphery of the connection mechanism; and
a first steering line and a second steering line,
wherein the fixing member is provided with at least one guide channel, and the first steering line and the second steering line share a same guide channel of the at least one guide channel, or the first steering line and the second steering line are arranged to pass through two guide channels of the at least one guide channel respectively;
wherein an end of the first steering line and an end of the second steering line are connected to the first connection portion and the second connection portion respectively, and another end of the first steering line and another end of the second steering line are connected to a corresponding part of the steering mechanism through a corresponding guide channel; and
wherein when the steering mechanism is operated to place the first steering line in a tensioned state, the second steering line is in a relaxed state; when the steering mechanism is operated to place the second steering line in a tensioned state, the first steering line is in a relaxed state, and in this manner, the connection mechanism is made to be rotatable in two opposite rotational directions relative to the fixing member to adjust a circumferential position of the connection mechanism and components associated with the connection mechanism.

20. A rotatably adjusted positioning device, comprising:
a fixing member,
a connection mechanism substantially extending in a longitudinal direction and rotatably connected to a distal side of the fixing member, wherein a single connection point is disposed on the connection mechanism, and
a single steering line,
wherein the fixing member is provided with a single guide channel;
wherein an end of the steering line is connected to an elastic reset mechanism wound around the single connection point, and another end is connected to the steering mechanism through the guide channel; and
wherein the steering mechanism is operated to place the steering line in a tensioned state to drive the connection mechanism rotate relative to the fixing member, and after the steering line is loosened, the elastic reset mechanism causes the connection mechanism to reversely rotate.
